Europäisches Patentamt

(19) European Patent Office

Office européen des brevets

(11) Veröffentlichungsnummer: **0 001 822**

**A2**

(12) **EUROPÄISCHE PATENTANMELDUNG**

(21) Anmeldenummer: 78101259.6

(22) Anmeldetag: 26.10.78

(51) Int. Cl.²: **A 61 K 9/48**
A 61 J 3/07, A 61 J 5/00

(30) Priorität: 03.11.77 GB 45755/77

(43) Veröffentlichungstag der Anmeldung:
16.05.79 Patentblatt 79/10

(84) Benannte Vertragsstaaten:
BE CH DE FR GB NL SE

(71) Anmelder: HOECHST AKTIENGESELLSCHAFT
Postfach 80 03 20
D-6230 Frankfurt/Main 80(DE)

(72) Erfinder: Walker, Stephen Ernest,Dr.
Chetwode Close 3
Buckingham(GB)

(72) Erfinder: Bedford, Keith
Broad Street 35
Newport Pagnell(GB)

(72) Erfinder: Eaves, Terence, Dr.
Parkway 16
Woburn Sands(GB)

(54) Verbesserte pharmazeutische Präparate in Form von festen Dosiereinheiten, Verfahren zu ihrer Herstellung sowie eine Kapselfüllmaschine.

(57) Verfahren zur Herstellung von pharmazeutischen Präparaten in Hartkapselform wobei man einen den Wirkstoff enthaltenden flüssigen Träger, der als wasserlösliche Schmelzmasse mit einem Erstarrungspunkt im Bereich zwischen 30 und 60°C oder als thixotropes Gel vorliegt, in eine zur Verabreichung als Dosiereinheit geeignete starre Hülle eindosiers. Kapselfüllmaschine, die mit einem Detektorsystem (12) und einem zur Dosierung von Flüssigkeiten geeigneten Abfüllkopf (23) ausgerüstet ist.

FIG.1

FIG.2

FIG.3

EP 0 001 822 A2

- 1 -

HOECHST AKTIENGESELLSCHAFT HOE 77/F 276

**Verbesserte pharmazeutische Präparate in Form von festen Dosiereinheiten, Verfahren zu ihrer Herstellung sowie eine Kapselfüllmaschine**

Die Erfindung betrifft die Herstellung pharmazeutischer Präparate in Hartkapselform, vorzugsweise in Hartgelatinekapselform.

Es ist wichtig, dass pharmazeutische Präparate eine bekannte Wirkstoffmenge enthalten, und in vielen Fällen ist eine genaue Dosierung erforderlich, insbesondere bei hochwirksamen, in geringen Mengen zu verabreichenden Arzneimitteln. Zur peroralen Verabreichung werden feste Dosiereinheitsformen im allgemeinen flüssigen Formen vorgezogen, da sie eine Verabreichung in genauer Dosis gestatten, im allgemeinen leichter zu verabreichen sowie häufig stabiler sind. Hartgelatinekapseln sind eine verhältnismässig teure, aber höchst annehmbare feste Dosiereinheitsform, wobei die Gelatinehüllen im allgemeinen mit Feststoff gefüllt werden. Die Verarbeitung von Feststoffen zu Granulaten und Pulvern zur Kapselfüllung stellt jedoch gewisse technische Probleme; beispielsweise ist es schwierig, eine einheitliche Dispersion des Wirkstoffs in den verwendeten Trägern bereitzustellen.

Vor kurzem wurde eine Kapselfüllmaschine beschrieben (DOS 26 12 472), womit pastenförmige oder halbfeste Stoffe in Hartgelatinekapselhüllen dosiert werden können. Der pastenförmige oder halbfeste Stoff wird als Schicht einheitlicher Dicke und Dichte extrudiert, und ein Rohrglied wird senkrecht durch die gesamte Dicke der Schicht eingeführt. Wegen der pastenartigen Konsistenz des zu dosierenden Stoffes verbleibt ein Pfropfen davon mit dem Glied und wird dann daraus in eine Kapselhülle ausgepresst und mittels Druckluft positioniert. Dieses System besitzt mehrere Nachteile: die Genauigkeit der Dosierung hängt davon ab, dass die erhaltene, extrudierte Stoffschicht eine sehr einheitliche Dicke besitzt, und die Probleme der Vermischung des Arzneimittels mit der pastenartigen Grundmasse müssen gelöst werden; das Rohrglied muss mit einem verhältnismässig komplizierten Mechanismus gesteuert werden, der das Glied aus seiner Stellung über der extrudierten Schicht in seine Stellung über der Kapselhülle schwenkt; das Ausstossen des Stoffpfropfens aus dem Glied ist schwierig; und bei Verwendung eines öligen Stoffes kann dieser gegebenenfalls an der Fuge der Kapsel ausfliessen.

Die Verwendung eines flüssigen Stoffes in Kapseln bringt Probleme, da die Flüssigkeit gegebenenfalls zwischen den Hälften der Kapselhülle ausfliesst. Dies ist vermeidbar, wenn man ein Band um die Fuge legt: dies führt jedoch zu einer zusätzlichen Stufe im Füllverfahren und erhöht daher die Herstellungskosten. In Weichgelatinekapseln kann man allerdings Flüssigkeiten verwenden. Weichgelatinekapseln vereinigen den Vorteil einer festen Dosiereinheitsform mit den Vorteilen einer Flüssigkeit im Hinblick darauf, dass eine einheitliche Vermischung des Wirkstoffs im Träger leicht zu erzielen ist und das so erhaltene Gemisch mit grosser Genauigkeit dosiert werden kann. Sie besitzen jedoch den Nachteil, dass hochspezialisierte Vorrichtungen zu ihrer Herstellung erforderlich sind, welche daher im allgemeinen eher von Unterlieferanten als von der pharmazeutischen Firma selbst durchgeführt wird.    Dies führt zu höheren Kosten als bei Hartgelatinekapseln und hat ferner weitere Nachteile, beispielsweise Probleme bezüglich Forschung und Entwicklung neuer Pharmazeutika.

Es sollte nicht nur die Wirkstoffmenge in einem pharmazeutischen Präparat bekannt sein, sondern auch der Grad und die Geschwindigkeit seiner Abgabe in vivo, das heisst seine biologische Verfügbarkeit.    Ueber die Formulierung von Präparaten mit hoher biologischer Verfügbarkeit des Wirkstoffs sowie in vielen Fällen spezifischen Abgabeeigenschaften bestehen Arbeiten.    Seit den 1960er Jahren wurden laufend Arbeiten über die Anwendung fester Lösungen, fester Dispersionen und aus dem Wirkstoff in einem geeigneten Träger, z.B. Harnstoff oder einem Polyäthylenglykol, bestehender eutektischer Gemische durchgeführt.    Es hat sich gezeigt (siehe beispielsweise Journal of Pharmaceutical Sciences 60 1281 (1971)), dass solche Systeme unter Verwendung von hydrophilen Trägern, z.B. Polyäthylenglykolen, zu einer herkömmlichen festen Formulierungen weit überlegenen biologischen Verfügbarkeit führen können, während man zur Verzögerung der Abgabe des wirksamen Prinzips hydrophobe Träger verwenden kann.    Als hydrophobe Grundmassen eignen sich unter anderem beispielsweise niedrigschmelzende Wachse, Oele sowie wasserunlösliche Polymere.    Im letzteren Fall kann das wasserunlösliche Polymer im Gemisch mit einem flüssigen hydrophilen Träger vorliegen:  es wurden Weichgelatinekapseln mit verzögerter Abgabe beschrieben, in welchen der flüssige Kern Polyäthylenglykol 600 und einige Prozent Polyvinylacetat enthält;  mit der Auflösung des Polyäthylenglykols in den Magensäften führt das vorhandene Wasser zur Fällung des Polyvinylacetats als Feststoff, wodurch die Abgabe des Wirkstoffs verzögert wird.  Mit zunehmendem Anteil an vorhandenem Polyvinylacetat nimmt die Abgabegeschwindigkeit des Wirkstoffs ab.

Der Nachteil bei der grosstechnischen Verwendung fester Dispersionen oder eutektischer Gemische besteht jedoch darin, dass sie allgemein sehr schlechte Verarbeitungseigenschaften besitzen, indem sie im allgemeinen klebrige glasartige Massen mit schlechtem Fliessverhalten darstellen, die mit der herkömmlichen Tablettier- oder Kapselfülltechnik sehr schwer zu verarbeiten sind.

Gegenstand vorliegender Erfindung ist ein Verfahren zur Herstellung pharmazeutischer Präparate in Form von Dosiereinheiten, welches dadurch gekennzeichnet ist, dass man einen den Wirkstoff enthaltenden flüssigen Träger in eine zur Verabreichung als Dosiereinheit geeignete starre Hülle, vorzugsweise in eine Hartgelatinekapsel, eindosiert, wobei die Flüssigkeit innerhalb der Hülle erstarrt oder soweit geliert, dass sie ihr flüssiges Fliessverhalten verliert; der flüssige Träger ist dabei eine wasserlösliche Schmelzmasse mit einem Erstarrungspunkt im Bereich zwischen 30 und 60°C, oder ein thixotropes Gel.

Zweckmässig ist die Hülle der Körper einer Hartgelatinekapsel.

Der den Wirkstoff enthaltende flüssige Träger kann beispielsweise ein thixotropes Gel sein, das sich während des Abfüllvorgangs wie eine Flüssigkeit, aber innerhalb der Kapselhülle wie ein Feststoff verhält, oder er kann eine wasserlösliche Schmelzmasse sein, die dann beim Abkühlen auf Raumtemperatur zu einer festen Lösung, einer festen Dispersion oder einem eutektischen Gemisch oder auch einem Gemisch dieser Formen erstarrt: zum Beispiel kann sich ein Arzneimittel teilweise in einem geschmolzenen Träger auflösen, so dass beim Erstarren ein Gemisch aus fester Lösung und fester Dispersion entsteht. Im allgemeinen liegt das Arzneimittel in der festen Dispersion als Einzelkristalle und nicht als Kristallaggregate wie in herkömmlichen Formulierungen vor, was die Auflösung unterstützt.

Bei Verwendung einer Schmelzmasse ist es wünschenswert, ein System mit einem Schmelzpunkt im Bereich von 30 bis 60°C und mit einer relativ niedrigen Viskosität im
geschmolzenen Zustand zu verwenden. Damit wird sichergestellt, dass sich die Schmelze ohne weiteres durch das
Kapselfüllsystem pumpen und im geschmolzenen Zustand
leicht rühren lässt, um die Auflösung sowie die Suspendierung irgendwelchen unlöslichen Materials zu fördern. Die
anwendbare Höchsttemperatur wird dadurch bestimmt, wie
stabil der Wirkstoff im geschmolzenen Träger ist, wie
leicht und wirtschaftlich der Kapselfüllapparat geheizt
werden kann und wie stabil die Kapselhüllen sind: eine
60°C übersteigende Temperatur kann eine herkömmliche Hartgelatinekapselhülle beschädigen, jedoch kann man bei der
Füllung sonstiger Hartkapseln höhere Temperaturen anwenden.

Geeignete wasserlösliche Träger sind beispielsweise Makrogolester wie Polyoxyl-40-stearat, Makrogoläther
wie Polyäthylenglykole, Poloxamer wie Poloxamer 188, Polyvinylalkoholsaccharoseester, zum Beispiel Crodesta F 160,
Carboxypolymethylen wie Carbopol, Sorbitanester wie Sorbitantrioleat, Polysorbate wie Polysorbat 80 oder irgendein Gemisch dieser.

Durch geeignete Auswahl des Trägermaterials kann
man die Auflösung des Arzneimittels in vivo fördern und
die biologische Verfügbarkeit des Arzneimittels erhöhen.
In einer festen Lösung oder Disperion oder einem eutektischen Gemisch liegt das Arzneimittel in einer sehr viel
feinverteilteren Form vor, als durch einfaches Vermischen
des Arzneimittels mit einem festen Träger erhältlich ist.
Ist der Träger der festen Lösung oder Dispersion bzw. des
eutektischen Gemischs in Magensäften sehr leicht löslich,
so löst er sich bei der Verabreichung sehr schnell auf,
und das Arzneimittel, das in sehr feinverteilter Form vorliegt, wird leicht und schnell resorbiert. Bei Arzneimitteln, die in Magensäften nicht sehr leicht löslich sind,
stellt dies einen grossen Vorteil dar. Polyäthylenglykole (PÄG) mit Molekulargewichten im Bereich von 1 000 bis
6 000 haben sich als Träger für derartige Schnellabgabesysteme bewährt; der Schmelzpunkt des Systems lässt sich durch Vermischen verschiedener PÄG-Sorten, beispielsweise PÄG 1000 mit einem Schmelzpunkt von etwa $35^{o}C$ und PÄG 6000 mit einem Schmelzpunkt von etwa $60^{o}C$, genau einregeln.

Ist der Träger dagegen in Magensäften nicht leicht löslich, so erhält man ein System mit verzögerter Abgabe; zu derartigen Trägern gehören mit Thixotropiemitteln gelierte Oele, zum Beispiel Paraffinöl. Eine effektive Formulierung mit verzögerter Abgabe lässt sich auch unter Verwendung von Polyvinylacetat wie oben beschrieben herstellen, jedoch unter Beimischung einer höheren Sorte PÄG, als bei Weichgelatinekapseln verwendet wurde, um eine feste Lösung in der Kapsel zu erzeugen.

Geeignete Geliermittel, die zur Herstellung thixotroper Gele zur Verfügung stehen, sind unter anderem hydriertes Rizinusöl (z.B. [R]Thixcin) und kolloidales Siliciumdioxyd (z.B. [R]Aerosil).

Da das erfindungsgemässe Verfahren einen flüssigen Träger verwendet, entfallen die Probleme einer einheitlichen Vermischung eines festen Arzneimittels mit einem festen Träger. Durch einfaches Rühren der Schmelzmasse bzw. des flüssigen Gels erhält man ein homogenes Gemisch bzw. eine homogene Lösung. Dies ist von besonderer Bedeutung bei Systemen, wo wirksames Vermischen von Feststoffen schwierig ist: zum Beispiel besitzen gewisse niedrig-dosierte perorale Empfängnisverhütungsmittel eine Teilchengrösse und Gestalt, welche die Herstellung fester Dosiereinheitsformen mit befriedigend einheitlichem Gehalt sehr erschweren. Es wurde zum Beispiel berichtet, dass der Steroidgehalt in niedrig-dosierten Empfängnisverhütungstabletten in ziemlich weiten Grenzen schwankt (vgl. von P.D. Faint und G.H. King am Technicon Colloquium über "Automated Analysis in the Pharmaceutical Industry [Automatisierte Analyse in der pharmazeutischen Industrie]", Bloomsbury Centre Hotel, London, 23. April 1970, gehaltener Vortrag). Solche Steroide füllt man daher sehr zweckmässig nach dem erfindungsgemässen Verfahren in Kapselhüllen ab. Das erfindungsgemässe Verfahren ist beispielsweise ebenfalls zur Herstellung von Digoxin enthaltenden Kapseln verwendbar; die bei dessen Auflösung auftretenden Probleme haben in jüngster Zeit erhebliches Interesse erweckt.

Ein grosser Vorteil des erfindungsgemässen Verfahrens besteht darin, dass man zur Füllung der Kapseln herkömmliche Hartgelatinekapselfüllmaschinen mit nur kleinem Umbau verwenden kann. Solche Maschinen zur Abfüllung von Pulvern in eine Kapselhülle besitzen einen Abfüllkopf, der einen Pulverpfropfen aufhebt und diesen in eine Kapselhülle fallen lässt, die sich auf einem Drehtisch befindet, welcher dann rotiert, um eine neue Hülle in die Abfüllstellung zu bringen. Durch Ersatz des Abfüllkopfes durch eine Vorrichtung zum Dosieren einer Flüssigkeit anstelle eines Pulvers lassen sich die Maschinen leicht zur Durchführung des erfindungsgemässen Verfahrens umbauen.

Gegenstand der Erfindung ist daher weiterhin eine Kapselfüllmaschine mit einem Drehtisch und Abfüllkopf, welche dadurch gekennzeichnet ist, dass die Maschine mit einem Detektorsystem und einem zur Dosierung von Flüssigkeiten geeigneten Abfüllkopf ausgerüstet ist. Als Abfüllkopf lassen sich beispielsweise eine nachfüllbare Spritze, eine Schlauchpumpe oder ein Präzisionsspritzgerät verwenden. Dabei hat sich ein mit Druckluft betätigtes Präzisionsspritzgerät besonders bewährt, da es eine exakte und wiederholbare Dosis Flüssigkeit liefert.

Wird eine Schmelzmasse verwendet, so sind Heizvorrichtungen erforderlich, um sicherzustellen, dass die Schmelze vor der Dosierung in die Kapselhülle nicht erstarrt oder zu viskos wird: die Temperatur der Schmelze bei der Dosierung in den Kapselhüllenkörper soll natürlich niedriger sein als die Temperatur, bei der die Hülle Schaden leiden würde. Ein Heizband um die entsprechenden Apparateteile stellt im allgemeinen die zweckmässigste Heizvorrichtung dar, jedoch sind andere Methoden, z.B. Heissluft, ebenfalls verwendbar.

In einer herkömmlichen Hartgelatinekapselfüllmaschine ist üblicherweise unter der Abfüllstation ein Aufnahmegefäss vorgesehen, damit das ausgegebene Material gesammelt wird, falls die Maschine keinen Kapselkörper unter den Abfüllkopf stellt. Wird jedoch die Maschine zur Ausgabe einer Flüssigkeit umgebaut, so ist dieses Sammelsystem unzureichend, da die Flüssigkeit den Apparat verschmutzt. Dies lässt sich durch Einbau eines Detektorsystems vermeiden, welches beispielsweise auf einer Photozelle beruht, so dass der Abfüllmechanismus nur dann Flüssigkeit ausgibt, wenn sich eine Kapselhülle in der richtigen Stellung unter dem Abfüllmechanismus befindet. Vorzugsweise wird dieses System erweitert, um die Gegenwart beschädigter Hüllen festzustellen und deren Abfüllung zu verhindern. Das Detektorsystem kann beispielsweise einen Mikroschalter betätigen, welcher den Drehtisch und den Abfüllmechanismus steuert; wenn keine Kapselhülle unter dem Abfüllmechanismus steht, wird vorzugsweise keine Flüssigkeit ausgegeben, und der Drehtisch rotiert, um die nächste Kapselhülle hinzustellen, so dass der Abfüllgang nicht ernsthaft unterbrochen wird.

Die Kapselfüllmaschine kann sowohl für intermittierende Bewegung als auch für kontinuierliche Bewegung ausgebildet sein.

Kapseln, die miteinander verträgliche Arzneimittel enthalten, kann man dadurch herstellen, dass man eine Flüssigkeit mit einem Arzneimittel in die Hülle dosiert und nach Erstarrung eine Flüssigkeit mit einem zweiten Arzneimittel in die Hülle dosiert. Ebenfalls können mit einer Schmelze und/oder einem Gel dosierte Hüllen auch Kügelchen, Granulate, Tabletten oder ein Pulver enthalten.

Somit bietet das erfindungsgemässe Verfahren die Vorteile einer Flüssigkeit im Hinblick auf einheitlichen Gehalt des entstandenen Produkts sowie die Vorteile eines Feststoffs im Hinblick auf bequeme Verabreichung und Stabilität des Präparats. Ferner lassen sich auch die Vorteile der Verwendung fester Lösungen, fester Dispersionen und eutektischer Gemische im Hinblick auf gesteuerte Abgabe des Arzneimittels und dessen biologische Verfügbarkeit ausnutzen. Ein weiterer bedeutender Vorteil besteht darin, dass eine für das erfindungsgemässe Verfahren verwendete Formulierung im allgemeinen sehr viel einfacher ist als eine bei der Verwendung herkömmlicher Tablettier- oder Kapselfülltechnik erforderliche Formulierung, wobei im allgemeinen die Gegenwart von verschiedener als Fliesshilfsmittel wirkenden Trägern, Schmiermitteln und Streckmitteln zur Verbesserung des Fliessverhaltens der Formulierung notwendig ist. Bei dem erfindungsgemässen Verfahren ist die flüssige Formulierung natürlich selbstschmierend und kann sehr einfach aufgebaut sein, beispielsweise aus einem Polyäthylenglykol und dem Arzneimittel oder einem Oel, einem Thixotropiemittel und dem Arzneimittel.

Die Erfindung sei nun anhand der beigefügten Zeichnungen nur beispielhaft beschrieben. Es zeigen:

Figur 1 den Drehtisch einer erfindungsgemässen Kapselfüllmaschine und deren dazugehörige Abfüllvorrichtung;

Figur 2 das Kapseldetektorsystem der Maschine in Figur 1; und

Figur 3 das in der Maschine in Figur 1 verwendete Präzisionsspritzgerät.

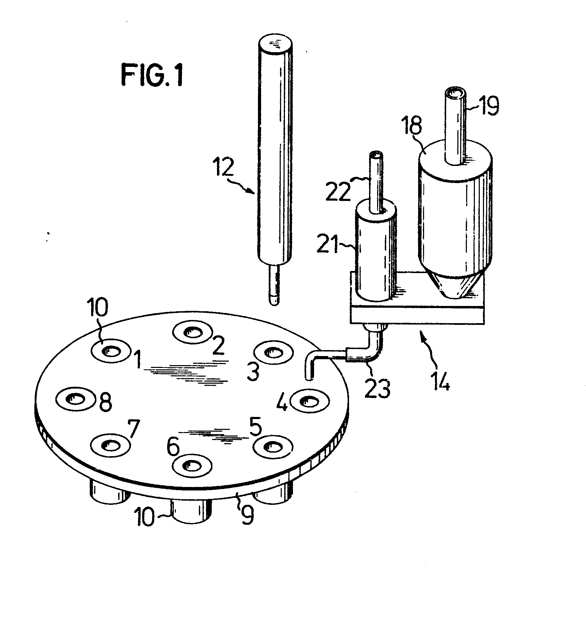

Figur 1 zeigt den Drehtisch 9 der Kapselfüllmaschine mit acht um deren Umfang angeordneten Stationen. Diese Stationen umfassen die Kapseleinführstation 1, Trennstation 2, Detektorstation 3, Abfüllstation 4, Kapselschliessstation 5, Auswerferstation 6, Regelstation 7 sowie eine Station 8, die beim erfindungsgemässen Verfahren nicht benutzt wird.

Bei der Kapseleinführstation 1 wird eine leere

Kapselhülle in einen Kapselhalter 10 auf dem Drehtisch 9 gestellt. Bei der Trennstation 2 wird der Kapseldeckel vom Kapselkörper 11 getrennt. Ueber der Detektorstation 3 ist ein Detektorsystem 12 angeordnet. Bei der Abfüllstation 4 wird flüssiges Füllmaterial 13 mittels eines Präzisionsspritzgeräts 14 in den Kapselkörper 11 eingespritzt. Bei Station 5 wird die Kapselhülle durch Wiederaufsetzen des Deckels auf den Kapselkörper 11 verschlossen, und die so erhaltene Kapsel wird bei der Auswerferstation 6 aus dem Drehtisch ausgeworfen. Das durch den Drehtisch 9 aktivierte Betätigungsglied (ein Mikroschalter, nicht dargestellt) für das Präzisionsspritzgerät 14 befindet sich an der Regelstation 7.

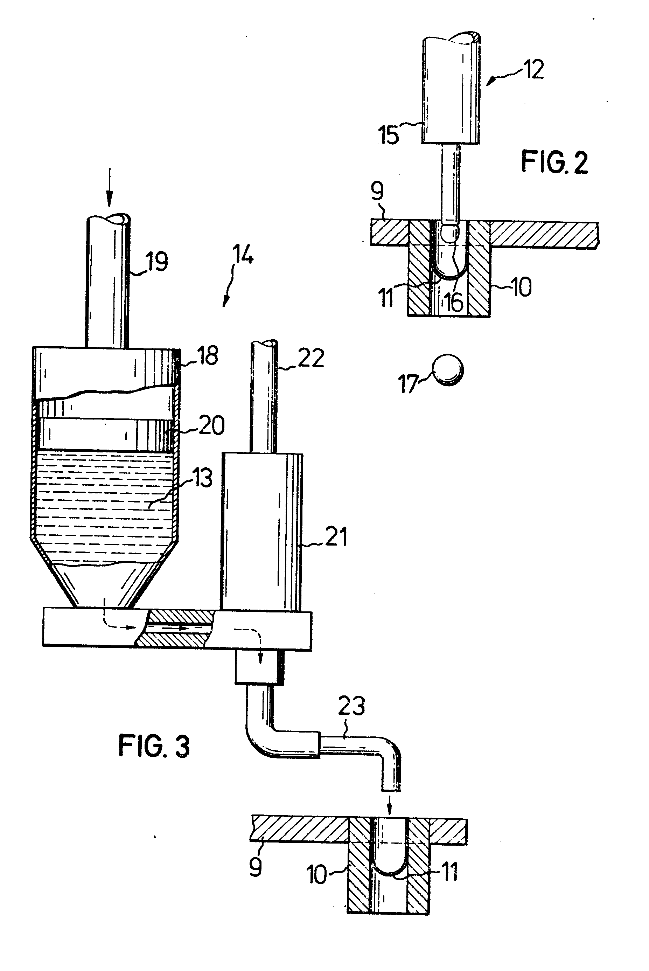

Das Detektorsystem 12 ist in Figur 2 mehr im einzelnen dargestellt. Es besteht aus einer Stützstange 15, die auf nicht dargestellte Weise in einer senkrechten Ebene beweglich ist, einer lichtemittierenden Diode 16 und einem Detektor 17. Ist im Kapselhalter 10 kein Kapselkörper 11 vorhanden, so erreicht Licht aus der lichtemittierenden Diode 16 den Detektor 17 und betätigt dadurch den Mikroschalter (nicht dargestellt) an der Steuerstation 7, was den Abfüllgang verzögert und somit ein Verschütten des flüssigen Füllmaterials 13 verhindert.

Das Präzisionsspritzgerät 14 ist in Figur 3 dargestellt. Es besteht aus einem Behälter 18 für flüssiges Füllmaterial 15, mit einem Einlass für Druckluft, die einen Kolben 20 betätigt; ein Ventilwerk 21, in dem das Ventil mittels durch einen Einlass 22 zugeführte Druckluft geöffnet oder geschlossen wird, ermöglicht, dass das flüssige Füllmaterial 13 in Richtung der Pfeile in eine Abfülldüse 23 und dann in den Kapselkörper 11 fliesst. Der Behälter 18 ist mit einer Heizvorrichtung (nicht dargestellt) versehen, beispielsweise einem Heizband oder einem Heissluftgebläse. Die abgegebene Menge flüssigen Füllmaterials 13 hängt vom am Einlass 19 angewandten Luftdruck und einem Zeitgeber (nicht dargestellt) ab, der von

dem Mikroschalter (nicht dargestellt) an der Regelstation 7 gesteuert wird. Ferner ist sie auch von Grössen wie der Viskosität des flüssigen Füllmaterials 13 abhängig.

Zur Durchführung des erfindungsgemässen Verfahrens kann das Präzisionsspritzgerät auch abgewandelt werden, zum Beispiel kann der Kolben 20 fehlen.

Die nachfolgenden Beispiele erläutern die Erfindung.

BEISPIEL 1:

| Formel | mg/Kapsel |
|---|---|
| Triamteren | 0,020 |
| Polyäthylenglykol (400) | 80,000 |
| Polyäthylenglykol (2000) | 320,000 |
| GESAMTFUELLGEWICHT | 400,020 mg |

Eine herkömmliche Kapselfüllmaschine, z.B. eine Höfliger- oder (R)Zanasimaschine, lässt sich dadurch zur Anwendung umbauen, dass man die normale Vorrichtung zur Dosierung eines Pulverpfropfens in die Kapselhülle durch eine nachfüllbare Spritze ersetzt. Ein Heissluftgebläse wurde vorgesehen, um den Abfüllmechanismus auf der erforderlichen Temperatur zu halten, und ein Detektorsystem unter Verwendung einer lichtemittierenden Diode wurde angebracht, um den Abfüllgang anzuhalten, falls aus irgendeinem Grund keine Kapselhülle zur rechten Zeit bei der nachfüllbaren Spritze erschienen war.

Das Polyäthylenglykolgemisch wurde bei 50°C geschmolzen und das Triamteren durch Rühren darin dispergiert. Stündlich wurden 3 600 Kapseln (Grösse 1) mit Schmelze gefüllt, und die relative Standard-Abweichung (RSA) des Füllgewichts betrug 2,7%. Demgegenüber zeigte eine Serie von stündlich 3 600 mit einem mittleren Füllgewicht von 269,23 mg einer herkömmlichen Pulverformulierung auf Basis von mikrokristalliner Zellulose, Streckmitteln, Magnesiumstearat und Talkum dosierten Kapseln eine relative Standard-Abweichung von 2,9%. Dies zeigt, dass die Einheitlichkeit des Füllgewichts der ähnlich ist, die man mit herkömmlichen Vorrichtungen erzielt. Die Einheitlichkeit des Arzneimittelgehalts in der Kapsel wurde ebenfalls bestimmt, und sogar bei einer niedrigen Dosis von 20 µg Triamteren war die relative Standard-Abweichung des Arzneimittels im Gemisch kleiner als 2%. Dies ist vergleichsweise gut gegenüber der Gehaltschwankung, die man bei nach herkömmlichen Tablettiermethoden zubereiteten, niedrig-dosierten empfängnisverhütenden Steroiden findet.

BEISPIEL 2:

| Formel | mg/Kapsel |
|---|---|
| Kolloidales Siliciumdioxyd (z.B. (R)AEROSIL 200) | 12,4 |
| Hydriertes Rizinusöl (z.B. (R)THIXCIN R) | 18,6 |
| Paraffinöl | 589,0 |
| GESAMTFUELLGEWICHT | 620 mg |

Das Thixcin und Aerosil werden unter Verwendung eines Schnellmischers im Paraffinöl dispergiert, und zur Entwicklung des Gels wird das Gemisch auf 40°C erwärmt. Das thixotrope Gel wird unter Verwendung eines Präzisionsspritzgeräts in Kapselhüllen abgefüllt. Für ein Füllgewicht von 620 mg lässt sich eine relative Standard-Abweichung von 1,4% erreichen.

BEISPIEL 3:

| Formel | mg/Kapsel |
|---|---|
| Triamteren | 50 |
| Polyäthylenglykol (400) | 70 |
| Polyäthylenglykol (2000) | 280 |
| GESAMTFUELLGEWICHT | 400 mg |

Methode

Das Triamteren wird unter Verwendung eines Dreiwalzenstuhls in Polyäthylenglykol 400 dispergiert, und geschmolzenes PÄG 2000 wird unter Rühren dazugegeben. Das geschmolzene Gemisch füllt man unter Verwendung einer nachfüllbaren Spritze in Kapseln ab.

- 13 -    0001822

Die Auflösung von gemäss dem erfindungsgemässen Verfahren gefüllten Triamterenkapseln und handelsüblichen Triamteren in 900 ml 0,1n-HCl nach der Methode des U.S. Arzneibuchs bei 100 U.p.m. ist in der Tabelle dargestellt:

Auflösung von 50 mg-Triamterenkapseln in 900 ml 0,1n-HCl bei 100 U.p.m. nach der U.S. Arzneibuchmethode

| | % Arzneimittelabgabe | |
| --- | --- | --- |
| Dosis | 50 mg | 50 mg |
| Dosierform | Handelsübliche Triamterenkapseln | Feste Lösung (Beispiel 3) |
| Auflösungszeit (Minuten) | | |
| 15 | 2 | 78 |
| 30 | 6 | 97 |
| 60 | 13 | 99 |
| 240 | 46 | – |
| 360 | 60 | – |
| Zerfallzeit nach dem britischen Arzneibuch (Minuten) | 30 | 9* |

*Der Kapselinhalt wird sehr schnell dispergiert; der grössere Teil dieser Zeit ist zur Auflösung der Kapselhülle nötig.

BEISPIEL 4:

| Formel | mg/Kapsel |
| --- | --- |
| Nomifensin-maleinsäurehalbester | 75 |
| Polyvinylacetat | 10- 30 (2-6 Gew./Vol.-%) |
| Polyäthylenglykol | 415-395 |
| GESAMTFUELLGEWICHT | 500 mg |

METHODE

Das PÄG-Gemisch wird bei 50°C geschmolzen und das Polyvinylacetat unter Rühren in der geschmolzenen Masse aufgelöst. Nomifensin-maleinsäurehalbester wird in der Schmelze dispergiert und das Ganze unter Verwendung einer nachfüllbaren Spritze in Kapselhüllen abgefüllt.

Derartige Formulierungen wirken mit verzögerter Abgabe: in dem Mass. wie Wasser mit dem Polyvinylacetat in Berührung kommt, erfolgt eine Fällung, wobei die Abgabe des Arzneimittels somit verlangsamt wird.

Auflösungsdaten sind in der Tabelle angegeben: Desaga-Durchflussauflösungsprüfung an Nomifensin - 75 mg mit Verzögerung; Einfluss der Polyvinylacetatkonzentration

| | % Arzneimittelabgabe | | |
|---|---|---|---|
| PVA-Konzentration % | 2 | 4 | 6 |
| Zeit (Stunden) | | | |
| 1,25 | 83 | 53 | 43 |
| 2,25 | 84 | 61 | 47 |
| 3,25 | 84 | 65,2 | 48 |
| 4,25 | – | 70,0 | 49 |
| 5,25 | – | 74,5 | 50 |

Die Auswirkung des Polyvinylacetatgehalts auf die Verzögerungsformulierung ist in der obigen Tabelle dargestellt. Man erkennt ein abgestuftes Ansprechen:

2% - hohe Abgabe im ersten Zeitabschnitt und keine Verzögerungswirkung

4% - befriedigende anfängliche Abgabe und allmähliche verzögerte Abgabe über die verbleibenden vier Stunden

6% - befriedigende anfängliche Abgabe mit wenig weiterer Arzneimittelabgabe über die verbleibenden vier Stunden.

0001822
HOE 77/F 276

Patentansprüche:

1.	Verfahren zur Herstellung von pharmazeutischen Präparaten in Dosiereinheitsform, dadurch gekennzeichnet, dass man einen den Wirkstoff enthaltenden flüssigen Träger in eine zur Verabreichung als Dosiereinheit geeignete starre Hülle eindosiert, wobei die Flüssigkeit innerhalb der Hülle erstarrt oder soweit geliert, dass sie ihr flüssiges Fliessverhalten verliert und wobei der flüssige Träger als wasserlösliche Schmelzmasse mit einem Erstarrungspunkt im Bereich zwischen 30 und 60°C, oder als thixotropes Gel vorliegt.

2.	Verfahren nach Anspruch 1, dadurch gekennzeichnet, dass der flüssige Träger aus einem Polyäthylenglykol mit einem Molekulargewicht im Bereich von 1 000 bis 6 000, einem Gemisch aus zwei oder mehreren solchen Polyäthylenglykolen oder einem Gemisch aus einem oder mehreren solchen Polyäthylenglykolen und Polyvinylacetat besteht.

3.	Verfahren nach Anspruch 1, dadurch gekennzeichnet, dass als thixotropes Geliermittel hydriertes Rizinusöl oder kolloidales Siliciumdioxyd vorliegt.

4.	Verfahren nach Anspruch 1, 2 oder 3, dadurch gekennzeichnet, dass die starre Hülle ein Hartgelatinekapselkörper ist.

5.	Kapselfüllmaschine mit einem Drehtisch und Abfüllkopf, dadurch gekennzeichnet, dass die Maschine mit einem Detektorsystem und einem zur Dosierung einer Flüssigkeit geeigneten Abfüllkopf ausgerüstet ist.

6.	Kapselfüllmaschine nach Anspruch 5, dadurch gekennzeichnet, dass der Abfüllkopf eine nachfüllbare Spritze, eine Schlauchpumpe oder ein Präzisionsspritzgerät ist.

7.	Verfahren nach einem der Ansprüche 1 bis 4, dadurch gekennzeichnet, dass der den Wirkstoff enthaltende flüssige Träger unter Verwendung einer Kapselfüllmaschine nach Anspruch 5 oder 6 in die starre Hülle eindosiert wird.

8.	Verfahren nach Anspruch 7, dadurch gekennzeichnet, dass die Dosierung in einer Hartgelatinekapselschnellfüllmaschine erfolgt.

9.   Verfahren nach Anspruch 7, durchgeführt im wesentlichen wie hier anhand der beigefügten Zeichnungen beschrieben.

10.   Pharmazeutische Zusammensetzung in Dosiereinheitsform, hergestellt gemäss dem Verfahren nach einem der Ansprüche 1 bis 4 und 7 bis 9.

0001822

FIG.1

FIG. 2

FIG. 3